(19) Europäisches Patentamt
European Patent Office
Office européen des brevets

(11) Veröffentlichungsnummer: **0 390 022 A2**

(12) **EUROPÄISCHE PATENTANMELDUNG**

(21) Anmeldenummer: **90105688.7**

(22) Anmeldetag: **26.03.90**

(51) Int. Cl.[5] **C07D 249/08, C07D 233/60, A01N 43/653, A01N 43/50**

(30) Priorität: **25.03.89 DE 3909862**

(43) Veröffentlichungstag der Anmeldung: **03.10.90 Patentblatt 90/40**

(84) Benannte Vertragsstaaten: **AT BE CH DE DK ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Seele, Rainer, Dr.**
**Leiblstrasse 3**
**D-6701 Fussgoenheim(DE)**
Erfinder: **Kober, Reiner, Dr.**
**Im Schlittweg 20**
**D-6701 Fussgoenheim(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**
Erfinder: **Jung, Johann, Prof. Dr.**
**Hardenburgstrasse 19**
**D-6703 Limburgerhof(DE)**
Erfinder: **Rademacher, Wilhelm, Dr.**
**Austrasse 1**
**D-6703 Limburgerhof(DE)**

(54) **Azolylethylcyclopropane, Verfahren zu ihrer Herstellung und ihre Verwendung als pflanzenschutzmittel.**

(57) Azolylethylcyclopropane der Formel I

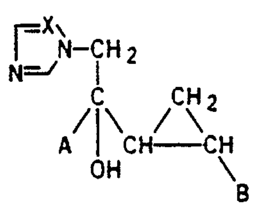

I,

in welcher
A und B gleich oder verschieden sind und jeweils $C_1$-$C_6$-Alkyl, $C_3$-$C_6$-Cycloalkyl, $C_3$-$C_6$-Cycloalkenyl, Tetrahydropyranyl, Pyridyl oder Phenyl bedeuten, wobei diese Reste einfach bis dreifach durch Halogen, Nitro, Phenoxy, Amino, Alkyl oder Halogenalkyl mit jeweils 1 bis 4 C-Atomen substituiert sein können, mit der Maßgabe, daß A und B nicht gleichzeitig Phenyl bedeuten,
X den Rest CH oder N bedeutet,
sowie deren für Pflanzen verträgliche Säureadditionssalze und Metallkomplexe und diese Verbindungen enthaltende Fungizide und Wachstumsregulatoren.

EP 0 390 022 A2

# Azolylethylcyclopropane, Verfahren zu ihrer Herstellung und ihre Verwendung als Pflanzenschutzmittel

Die vorliegende Erfindung betrifft neue Azolverbindungen, Verfahren zu ihrer Herstellung und diese enthaltende Fungizide und Wachstumsregulatoren.

Es ist bekannt, E-1-[-1-(4-chlorphenyl)-1-hydroxy2-(1,2,4-triazol-1-yl)-1-ethyl]-2-phenyl-cyclopropan (EP 212 605) als Fungizid und Wachstumsregulator zu verwenden. Die fungiziden und wachstumsregulatorischen Wirkungen sind jedoch nicht in allen Fällen befriedigend.

Es wurde nun gefunden, daß Verbindungen der Formel I

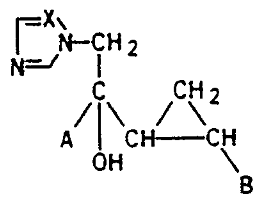

I,

in welcher

A und B gleich oder verschieden sind und jeweils $C_1$-$C_6$-Alkyl, $C_3$-$C_5$-Cycloalkyl, $C_3$-$C_6$-Cycloalkenyl, Tetrahydropyranyl, Pyridyl oder Phenyl bedeuten, wobei diese Reste einfach bis dreifach durch Halogen, Nitro, Phenoxy, Amino, Alkyl oder Halogenalkyl mit jeweils 1 bis 4 C-Atomen substituiert sein können, mit der Maßgabe, daß A und B nicht gleichzeitig Phenyl bedeuten,

X den Rest CH oder H bedeutet,

sowie deren für Pflanzen verträgliche Säureaddidionssalze oder Metallkomplexe eine bessere fungizide und wachstumsregulatorische Wirkung besitzen als bekannte Azolverbindungen.

Die Verbindungen der Formel I enthalten chirale Zentren und werden im allgemeinen in Form von Diastereomerengemischen erhalten. Die Diastereomeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Aus einem solchen isolierten Diastereomeren kann man mit bekannten Methoden einheitliche Enantiomere erhalten. Als fungizide und wachstumsregulatorische Mittel kann man sowohl die einheitlichen Diastereomere bzw. Enantiomere wie auch deren bei der Synthese anfallenden Gemische verwenden.

Alle diese Verbindungen und ihre Gemische werden von der vorliegenden Erfindung erfaßt.

A bzw. B bedeuten beispielsweise Methyl, Ethyl, Isopropyl, n-Propyl, n-Butyl, sec-Butyl, tert.-Butyl, n-Pentyl, Neopentyl, Hexyl, Trifluormethyl, Trichlormethyl, Phenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, 2-Methoxyphenyl, 3-Methoxyphenyl, 4-Methoxyphenyl, 2,4-Dimethoxyphenyl, 4-Ethylphenyl, 4-Isopropylphenyl, 4-tert.-Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Trifluoromethylphenyl, 3-Trifluoromethylphenyl, 4-Trifluoromethylphenyl, Cyclopropyl, Cyclobutyl, Cyclopentyl, Cyclohexyl, Tetrahydropyranyl, 2-Cyclohexenyl, 3-Cyclohexenyl, Pyridyl.

Säureadditionssalze sind beispielsweise die Hydrochloride, Bromide, Sulfonate, Nitrate, Phosphate, Oxalate oder Dodecylbenzolsulfonate. Die Wirksamkeit der Salze geht auf das Kation zurück, so daß es auf das Anion i.a. nicht ankommt. Die erfindungsgemäßen Wirkstoffsalze werden hergestellt durch Umsetzung der Azolylethylcyclopropane (I) mit geeigneten Säuren.

Metallkomplexe der Wirkstoffe I oder ihrer Salze können z.B. mit Kupfer, Zink, Zinn, Mangan, Eisen, Kobalt oder Nickel gebildet werden, indem man die Azolylethylcyclopropane mit entsprechenden Metallsalzen umsetzt, z.B. mit Kupfersulfat, Zinkchlorid, Zinnchlorid, Mangansulfat, Eisensulfat.

Die Verbindungen der Formel I können hergestellt werden, indem man eine Verbindung der Formel II

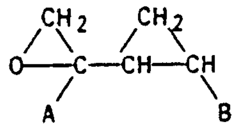

II,

in welcher
A und B die oben angegebene Bedeutung haben mit einer Verbindung der Formel III

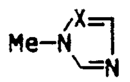

III,

in der
Me ein Wasserstoffatom oder ein Metallatom bedeutet und
X die oben angegebene Bedeutung hat,
zur Umsetzung bringt.

Die Reaktion erfolgt - falls Me ein Wasserstoffatom bedeutet - z.B. gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120 °C. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium-, Kalium- oder Caesiumcarbonat oder Natrium, Kalium- oder Caesiumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumjodid, oder Kaliumjodid, quaternäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-5, 18-Krone-6, Dibenzo-18-krone-6 oder Dicyclohexano-18-krone-6 in Frage.

Die Umsetzung wird im allgemeinen bei Temperaturen zwischen 20 und 150 °C, vorzugsweise zwischen 20 und 120 °C, drucklos oder unter Druck, kontinuierlich oder diskontinuierlich durchgeführt.

Ist Me ein Metallatom wird die Reaktion gegebenenfalls in Gegenwart eines Lösungs- oder Verdünnungsmittels und gegebenenfalls unter Zusatz einer starken anorganischen oder organischen Base im allgemeinen bei Temperaturen zwischen -10 und 120 °C durchgeführt. Zu den bevorzugten Lösungs- und Verdünnungsmitteln gehören Amide wie Dimethylformamid, Diethylformamid, Dimethylacetamid, Diethylacetamid, N-Methylpyrrolidon, Hexamethyl-phosphortriamid, Sulfoxide wie Dimethylsulfoxid und schließlich Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert.-butoxid, Lithium-, Natrium- oder Kalium-triphenylmethyl und Naphthalinlithium, -natrium oder -kalium.

Die Ausgangsverbindungen II können z.B. hergestellt werden, indem man ein ungesättigtes Keton der Formel IV

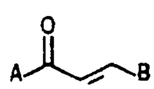

IV,

in welcher

A und B die oben angegebene Bedeutung haben, mit zwei Äquivalenten des Schwefelylids V

$$(CH_3)_2\overset{O}{\overset{\|}{S}}=CH_2 \qquad V$$

umsetzt (vgl. Corey, Chaykovsky, J. Am. Chem. Soc. 1962, 64, 3782).

Die Verbindungen der Formel IV lassen sich entsprechend allgemein bekannter Verfahren zur Olefinsynthese (vgl. z.B. Houben-Weyl-Müller, Methoden der organischen Chemie, Georg Thieme Verlag, Stuttgart 1972, Bd. V, 1b) herstellen.

Die folgenden Beispiele erläutern die Herstellung der Wirkstoffe.

1. Herstellung der Ausgangsstoffe

Beispiel A

Zu einer Lösung von 50g Pinakolon in 200 ml Ethanol und 1 ml Natronlauge (50gew. %ig) werden langsam 87,5 g 2,4-Dichlorbenzaldehyd gegeben, wobei die Innentemperatur 30 °C nicht übersteigt. Nach 24stündigem Rühren bei Raumtemperatur (20 °C) wird der entstandene Niederschlag abgesaugt und anschließend getrocknet. Man erhält 105,5 g (82 %) Tert.-butyl-(2,4-dichlorstyryl)-keton.

Beispiel B

90 g Tert.-butyl-(2,4-dichlorstyryl)-keton werden in 200 ml Dimethylformamid gelöst und bei 0 °C unter Stickstoffatmosphäre mit 86,9 g Trimethylsulfoxoniumjodid versetzt. Anschließend wird schnell 44,2 g Kalium-tert.-butylat zugegeben und auf 60 °C erhitzt. Nachdem das Reaktionsgemisch drei Tage bei 60 °C rührte, wird der Lösung 200 ml Wasser zugesetzt und mehrmals mit Methyl-tert.-butylether ausgeschüttelt. Die isolierte organische Phase wird zweimal mit Wasser gewaschen, daraufhin über Natriumsulfat getrocknet und eingeengt, wobei 69,8 g (70 %) Trans-1-[2-Tert.-butyl-oxiran-2-yl]2-(2,4-dichlorphenyl)-cyclo-propan erhalten werden.

II. Herstellung der Endprodukte

Beispiel 1

Eine Lösung von 5,1 g Triazol in 50 ml N-Methylpyrrolidon wird mit 5,6 g Natriumhydroxid (50gew.%ig) versetzt und für 30 Minuten auf 50 °C erhitzt. Anschließend wird bei Raumtemperatur 10 g Trans-1-(2-Tert-butyl-oxiran-2-yl]-2-(2,4-dichlorphenyl)-cyclopropan , das in 50 ml N-Methylpyrrolidon gelöst ist, langsam zugetropft. Nachdem das Reaktionsgemisch 18 Stunden bei Raumtemperatur rührte, wird der Lösung 100 ml Wasser zugesetzt und mehrmals mit Methyl-tert.-butylether ausgeschüttelt. Die isolierte organische Phase wird zweimal mit Wasser gewaschen, daraufhin über Natriumsulfat getrocknet und eingeengt. Der verbleibende Rückstand wird durch Flash-Chromatographie an Kieselgel (Essigester/n-Hexan = 9 : 1) gereinigt, wobei die getrennten Diastereo meren erhalten werden. Man erhält 7,4 g (60 %, Gesamtausbeute) Trans-1-[1-Tert.-butyl-1-hydroxy-2-(1,2,4-triazol-1-yl)-1-ethyl]-2-(2,4-dichlorphenyl)-cyclopropan.

Diastereomer A; Fp.: 151 - 158 °C (Verbindung Nr. 1)

Diastereomer B; Fp.: 98 - 101 °C (Verbindung Nr. 2)

Entsprechend Beispiel 1 können die in der Tabelle aufgeführten Verbindungen hergestellt werden.

Tabelle

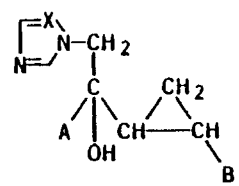

| Bsp.-Nr. | A | B | X | Schmp/IR | Isomer |
|---|---|---|---|---|---|
| 1 | tert.-$C_4H_9$ | 2,4-$Cl_2$-$C_6H_3$ | N | 151-153°C | Diastereomer 1 |
| 2 | tert.-$C_4H_9$ | 2,4-$Cl_2$-$C_6H_3$ | N | 98-101°C | Diastereomer 2 |
| 3 | tert.-$C_4H_9$ | 2,4-$Cl_2$-$C_6H_3$ | N | 163-167°C | $D_1 : D_2 = 3 : 2$ |
| 4 | tert.-$C_4H_9$ | 2-Cl-$C_6H_4$ | N | | |
| 5 | tert.-$C_4H_9$ | 2-Cl-$C_6H_4$ | CH | | |
| 6 | tert.-$C_4H_9$ | 4-Cl-$C_6H_4$ | N | 193-198°C | Diastereomer 1 |
| 7 | tert.-$C_4H_9$ | 4-Cl-$C_6H_4$ | N | 111-113°C | Diastereomer 2 |
| 8 | tert.-$C_4H_9$ | 4-Cl-$C_6H_4$ | CH | 147-148°C | $D_1 : D_2 = 2 : 1$ |
| 9 | tert.-$C_4H_9$ | 2-Cl-4-F-$C_6H_3$ | N | | |
| 10 | tert.-$C_4H_9$ | 2-$CF_3$-$C_6H_4$ | N | | |
| 11 | tert.-$C_4H_9$ | 4-$CF_3$-$C_6H_4$ | N | | |
| 12 | tert.-$C_4H_9$ | 2-F-$C_6H_4$ | N | | |
| 13 | tert.-$C_4H_9$ | 4-F-$C_6H_4$ | N | | |
| 14 | tert.-$C_4H_9$ | 2,4-$F_2$-$C_6H_3$ | N | | |
| 15 | tert.-$C_4H_9$ | 2-Br-$C_6H_4$ | N | | |
| 16 | tert.-$C_4H_9$ | 4-Br-$C_6H_4$ | N | | |
| 17 | tert.-$C_4H_9$ | $C_6H_5$ | N | | |
| 18 | tert.-$C_4H_9$ | 2-$C_{10}H_7$ | N | | |
| 19 | tert. $C_4H_9$ | 4-$C_{12}H_9$ | N | | |

Tabelle (Fortsetzung)

| Bsp.-Nr. | A | B | X | Schmp/IR | Isomer |
|---|---|---|---|---|---|
| 20 | tert.-$C_4H_9$ | 2-$CH_3$-$C_6H_4$ | N | | |
| 21 | tert.-$C_4H_9$ | 2-$OCH_3$-$C_6H_4$ | N | 131-135°C | Diastereomer 1 |
| 22 | tert.-$C_4H_9$ | 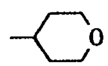 | N | | |
| 23 | $CH_3$ | 2-Cl-$C_6H_4$ | N | | |
| 24 | $CH_3$ | 4-Cl-$C_6H_4$ | N | | |
| 25 | $CH_3$ | 2,4-$Cl_2$-$C_6H_3$ | N | | |
| 26 | $CH_3$ | 2-$CF_3$-$C_6H_4$ | N | | |
| 27 | $CH_3$ | 2-Br-$C_6H_4$ | N | | |
| 28 | Cyclopropyl | 2-Cl-$C_6H_4$ | N | | |
| 29 | Cyclopropyl | 3-Cl-$C_6H_4$ | N | | |
| 30 | Cyclopropyl | 4-Cl-$C_6H_4$ | N | 108-110°C | $D_1 : D_2 = 6 : 1$ |
| 31 | Cyclopropyl | 4-Cl-$C_6H_4$ | N | Harz | $D_1 : D_2 = 3 : 2$ |
| 32 | Cyclopropyl | 4-Cl-$C_6H_4$ | CH | | |
| 33 | Cyclopropyl | 2,4-$Cl_2$-$C_6H_3$ | N | | |
| 34 | Cyclopropyl | 4-F-$C_6H_4$ | N | | |
| 35 | Cyclopropyl | 2-$CF_3$-$C_6H_4$ | N | | |

Tabelle (Fortsetzung)

| Bsp.-Nr. | A | B | X | Schmp/IR | Isomer |
|---|---|---|---|---|---|
| 36 | Cyclopropyl | 4-$NO_2$-$C_6H_4$ | N | | |
| 37 | Cyclopropyl | 4-$NH_2$-$C_6H_4$ | N | | |
| 38 | Cyclopropyl | p-$C_6H_5$-O-$C_6H_4$ | N | | |
| 39 | Cyclopropyl | 2-$C_{10}H_7$ | N | | |
| 40 | Cyclohexyl | 2-Cl-$C_6H_4$ | N | | |
| 41 | Cyclohexyl | 3-Cl-$C_6H_4$ | N | | |
| 42 | Cyclohexyl | 4-Cl-$C_6H_4$ | N | 138-140°C | $D_1 : D_2 = 3 : 1$ |
| 43 | Cyclohexyl | 2-F-$C_6H_4$ | N | | |
| 44 | Cyclohexyl | 2-$CF_3$-$C_6H_4$ | N | | |
| 45 | $C_6H_5$ | Cyclopropyl | N | | |
| 46 | $C_6H_5$ | Cyclopropyl | N | | |
| 47 | $C_6H_5$ | 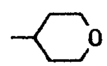 | N | | |
| 48 | 4-Cl-$C_6H_4$ | Cyclopropyl | N | | |
| 49 | 4-Cl-$C_6H_4$ | Cyclohexyl | N | | |
| 50 | 4-Cl-$C_6H_4$ | 2-Cyclohexenyl | N | | |
| 51 | 4-Cl-$C_6H_4$ | 3-Cyclohexenyl | N | | |
| 52 | 4-Cl-$C_6H_4$ | 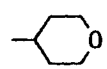 | N | | |
| 53 | 2,4-$Cl_2$-$C_6H_3$ | Cyclopentyl | N | | |
| 54 | 2,4-$Cl_2$-$C_6H_3$ | Cyclohexyl | N | | |

Tabelle (Fortsetzung)

| Bsp.-Nr. | A | B | X | Schmp/IR | Isomer |
|---|---|---|---|---|---|
| 55 | 4-F-$C_6H_4$ | Cyclopropyl | N | | |
| 56 | 4-F-$C_6H_4$ | Cyclopentyl | N | | |
| 57 | 4-F-$C_6H_4$ | Cyclohexyl | N | | |
| 58 | 4-F-$C_6H_4$ | 2-Cyclohexenyl | N | | |
| 59 | 4-$OCH_3$-$C_6H_4$ | Cyclopropyl | N | | |
| 60 | 4-$OCH_3$-$C_6H_4$ | Cyclohexyl | N | | |
| 61 | 4-$OCH_3$-$C_6H_4$ | 3-Cyclohexenyl | N | | |
| 62 | 4-$OCH_3$-$C_6H_4$ | $CCl_3$ | N | | |
| 63 | 4-$OCH_3$-$C_6H_4$ | $CCl_3$ | CH | | |
| 64 | 3-Pyridyl | tert.-$C_4H_9$ | N | 132-134°C | $D_1 : D_2 = 4 : 1$ |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die Aufwandmengen der fungiziden Mittel liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die neuen Verbindungen können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation),

d) von klimatischen Faktoren, z.B. Temperatur, Niederschlagsmenge, außerdem auch Tageslänge und Lichtintensität,

e) von der Bodenbeschaffenheit (einschließlich Düngung),

f) von der Formulierung bzw. Anwendungsform des Wirkstoffs und

g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Reis, Mais, Sonnenblumen und Soja. Die dabei verursachte Halmverkürzung und Halmverstärkung verringern oder beseitigen die Gefahr des "Lagerns" (des Um knickens) von Pflanzen unter ungünstigen Witterungsbedingungen vor der Ernte.

Von praktischer Bedeutung ist auch die Reduzierung des vegetativen Wachstums bei Obstbäumen und anderen Gehölzen, da so Schnittkosten eingespart werden können.

Wichtig ist auch die Anwendung zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung der neuen Verbindungen kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit den neuen Verbindungen läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.

B. Mit den neuen Mitteln lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Die Azolylethylcyclopropane der Formel I können Ertragssteigerungen durch Eingriffe in den pflanzlichen Stoffwechsel bzw. durch Förderung oder Hemmung des vegetativen und/oder des generativen Wachstums verursachen.

C. Mit den erfindungsgemäßen Verbindungen der Formel I lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Die Förderung der Ausbildung eines Trenngewebes zwischen der Blatt-und Sproßachse ist auch für ein gut kontrollierbares Entblättern von Nutzpflanzen, z.B. Baumwolle, wesentlich.

D. Mit den Azolylethylcyclopropanen kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie durch Spritzung über das Blatt zugeführt werden.

Infolge der hohen Verträglichkeit der Pflanzen für die Verbindungen I kann die Aufwandmenge als Wachstumsregulatoren stark variiert werden. Bei der Saatgutbehandlung werden im allgemeinen Wirkstoffmengen von 0,001 bis 50 g je Kilogramm Saatgut, vorzugsweise 0,005 bis 0,5 g, benötigt. Für die Blatt- und Bodenbehandlung sind im allgemeinen Gaben von 0,01 bis 10 kg/ha, bevorzugt 0,05 bis 1 kg/ha ausreichend.

Die Mittel auf der Basis von Azolylethylcyclopropanen können in Form üblicher Formulierungen

angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylole, Toluol), chlorierte Aromaten (z.B. Chlorbenzole), Paraffine (z.B. Erdölfraktionen), Alkohole (z.B. Methanol, Butanol), Amine (z.B. Ethanolamin), N,N-Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel oder sonstige oberflächenaktive Mittel, wie nichtionogene und anionische Emulgatoren (z.B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung gegebenenfalls unter Zusatz von mit Wasser mischbaren organischen Lösungsmitteln wie Methanol oder anderen niederen Alkoholen, Aceton, N,N-Dimethylformamid oder N-Methylpyrrolidon.

Die fungiziden und wachstumsregulierenden Mittel enthalten im allgemeinen zwischen 0,1 und 95, vorzugsweise zwischen 0,5 und 90 Gew.% Wirkstoff.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. S mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 2 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 2 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 2 werden mit 3 Gew.-Teilen des Natriumsalzes der Diisobutylnaphthalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 2 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels ge sprüht wurde, innig vermischt. Man erhält auf diese Weise eine Aufbereitung des Wirkstoffs mit guter Haftfähigkeit.

VIII. 40 Gew.-Teile der Verbindung Nr. 2 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 2 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums. Beim Vermischen mit Wachstumsregulatoren treten ebenfalls häufig synergistische Effekte auf.

Anwendungsbeispiele

Als Vergleichswirkstoff wurde E-1-[-1-(4-Chlorphenyl)-1-hydroxy-2-(1.2.4-triazol-1-yl)-1-ethyl]-2-phenyl - cyclopropan (A) - bekannt aus EP 212 605 - benutzt.

Anwendungsbeispiel 1

Wirksamkeit gegen Botrytis cinerea an Paprika

Paprikasämlinge der Sorte "Neusiedler Ideal Elite" wurden, nachdem sich 4 bis 5 Blätter gut entwickelt hatten, mit wäßrigen Suspensionen, die 80% Wirkstoff und 20% Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und in eine Kammer mit hoher Luftfeuchtigkeit und einer Temperatur von 22 bis 24°C gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß die Wirkstoffe 3, 6 und 31 bei der Anwendung als 0,05gew.%ige Spritzbrühe eine bessere fungizide Wirkung (95 %) zeigen als der bekannte Vergleichswirkstoff A (40 %).

Anwendungsbeispiel 2

Wirksamkeit gegen Pyricularia oryzae (protektiv)

Blätter von in Töpfen gewachsenen Reiskeimlingen der Sorte "Bahia" wurden mit wäßrigen Emulsionen, die 80% Wirkstoff und 20% Emulgiermittel in der Trockensubstanz enthielten, tropfnaß besprüht und 24 Stunden später mit einer wäßrigen Sporensuspension von Pyricularia oryzae inoculiert. Anschließend wurden die Versuchspflanzen in Klimakammern bei 22 bis 24°C und 95 bis 99% relativer Luftfeuchtigkeit aufgestellt. Nach 6 Tagen wurde das Ausmaß des Krankheitsbefalls ermittelt.

Das Ergebnis zeigt, daß die Wirkstoffe 6 und 8 bei der Anwendung als 0,05gew.%ige Spritzbrühe eine bessere fungizide Wirkung (90 %) zeigen als der bekannte Vergleichswirkstoff A (65 %).

Zur Bestimmung der wachstumsregulierenden Eigenschaften der Prüfsubstanzen wurden Testpflanzen auf ausreichend mit Nährstoffen versorgtem Kultursubstrat in Kunststoffgefäßen (ca. 12,5 cm Durchmesser; Volumen ca. 500 ml) angezogen.

Im Vorauflaufverfahren wurden die Testsubstanzen in wäßriger Aufbereitung am Tage der Einsaat auf das Saatbett gegossen.

Im Nachauflaufverfahren wurden die zu prüfenden Substanzen in wäßriger Aufbereitung auf die Pflanze gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanzen dienten der bekannte Wirkstoff Chlorcholinchlorid (B) und der Wirkstoff gemäß Beispiel 15 von EP-212 605 (A).

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzelheiten sind den folgenden Tabellen zu entnehmen:

Vergleichssubstanzen:

B

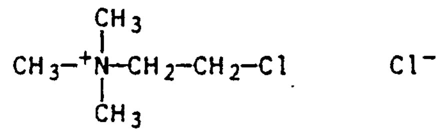

A

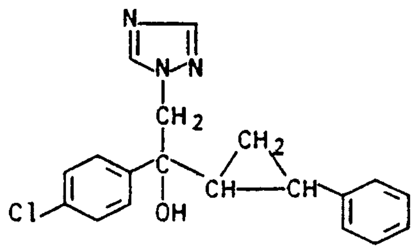

| Anwendungsbeispiel 3 | | |
|---|---|---|
| Sommergerste Sorte "Aramir"<br>Vorauflauf-Bodenbehandlung | | |
| Wirkstoff Nr. | Konzentration mg Wirkstoff/Gefäß | Wuchshöhen relativ |
| unbehandelt | - | 100 |
| B | 6 | 80,0 |
| A | 6 | 94,7 |
| 1 | 6 | 45,8 |
| 6 | 6 | 42,5 |
| 30 | 6 | 39,2 |
| 31 | 6 | 55,5 |

| Anwendungsbeispiel 4 | | |
|---|---|---|
| Sommerraps Sorte "Petranova"<br>Vorauflauf-Bodenbehandlung | | |
| Wirkstoff Nr. | Konzentration mg Wirkstoff/Gefäß | Wuchshöhen relativ |
| unbehandelt | - | 100 |
| B | 6 | 92,7 |
| A | 6 | 100,0 |
| 1 | 6 | 18,1 |
| 3 | 6 | 76,6 |
| 6 | 6 | 26,2 |
| 8 | 6 | 74,6 |
| 30 | 6 | 18,2 |
| 31 | 6 | 24,2 |

| Anwendungsbeispiel 5 | | |
|---|---|---|
| Sommerraps Sorte "Petranova" Nachauflauf-Blattbehandlung | | |
| Wirkstoff Nr. | Konzentration mg Wirkstoff/Gefäß | Wuchshöhen relativ |
| unbehandelt | - | 100 |
| B | 6 | 93,2 |
| A | 6 | 85,0 |
| 1 | 6 | 60,8 |
| 2 | 6 | 74,9 |
| 3 | 6 | 74,9 |
| 6 | 6 | 64,8 |
| 8 | 6 | 72,9 |

## Ansprüche

1. Azolylethylcyclopropane der Formel I

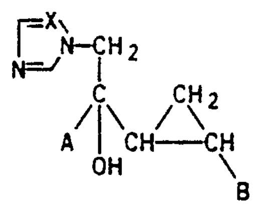

I,

in welcher

A und B gleich oder verschieden sind und jeweils $C_1$-$C_6$-Alkyl, $C_3$-$C_6$-Cycloalkyl, $C_3$-$C_6$-Cycloalkenyl, Tetrahydropyranyl, Pyridyl oder Phenyl bedeuten, wobei diese Reste einfach bis dreifach durch Halogen, Nitro, Phenoxy, Amino, Alkyl oder Halogenalkyl mit jeweils 1 bis 4 C-Atomen substituiert sein können, mit der Maßgabe, daß A und B nicht gleichzeitig Phenyl bedeuten,

X den Rest CH oder N bedeutet,

sowie deren für Pflanzen verträgliche Säureadditionssalze und Metallkomplexe.

2. Azolylethylcyclopropane der Formel I gemäß Anspruch 1, in denen A die tertiäre Butylgruppe bedeutet und B die Phenylgruppe bedeutet, welche unsubstituiert ist oder durch einen oder zwei Substituenten substituiert ist, die Fluor, Chlor, Brom oder die Trifluormethylgruppe bedeuten.

3. Verfahren zur Herstellung der Azolylethylcyclopropane der Formel 1 gemäß Anspruch 1, dadurch gekennzeichnet, daß man eine Verbindung der Formel II

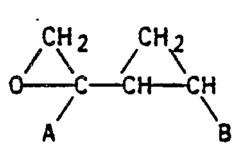

II,

in welcher

A und B die oben angegebene Bedeutung haben mit einer Verbindung der Formel III

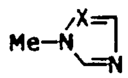

III,

in der
Me ein Wasserstoffatom oder ein Metallatom bedeutet und
X die oben angegebene Bedeutung hat,
umsetzt und die so erhaltenen Verbindungen gegebenenfalls in ihre Salze mit für Pflanzen verträglichen Säuren oder Metallkomplexen überführt.

4. Fungizides Mittel, enthaltend einen Trägerstoff und ein Azolylethylcyclopropan der Formel I

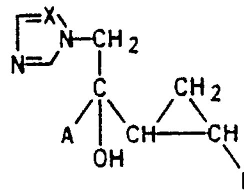

I,

in welcher
A und B gleich oder verschieden sind und jeweils $C_1$-$C_6$-Alkyl, $C_3$-$C_6$-Cycloalkyl, $C_3$-$C_6$-Cycloalkenyl, Tetrahydropyranyl, Pyridyl oder Phenyl bedeuten, wobei diese Reste einfach bis dreifach durch Halogen, Nitro, Phenoxy, Amino, Alkyl oder Halogenalkyl mit jeweils 1 bis 4 C-Atomen substituiert sein können, mit der Maßgabe, daß A und B nicht gleichzeitig Phenyl bedeuten,
X den Rest CH oder N bedeutet,
oder dessen für Pflanzen verträgliches Säureadditionssalz und Metallkomplex.

5. Verfahren zur Bekämpfung von Pilzen, dadurch gekennzeichnet, daß man eine fungizid wirksame Menge eines Azolylethylcyclopropans der Formel I

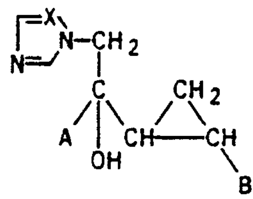

I,

in welcher
A und B gleich oder verschieden sind und jeweils $C_1$-$C_6$-Alkyl, $C_3$-$C_6$-Cycloalkyl, $C_3$-$C_6$-Cycloalkenyl, Tetrahydropyranyl, Pyridyl oder Phenyl bedeuten, wobei diese Reste einfach bis dreifach durch Halogen, Nitro, Phenoxy, Amino, Alkyl oder Halogenalkyl mit jeweils 1 bis 4 C-Atomen substituiert sein können, mit der Maßgabe, daß A und B nicht gleichzeitig Phenyl bedeuten,
X den Rest CH oder N bedeutet,
oder dessen für Pflanzen verträgliche Säureadditionssalze oder Metallkomplexe auf die Pilze oder durch Pilzbefall bedrohte Materialien, Flächen, Pflanzen oder Saatgüter einwirken läßt.

6. Wachstumsregulatorisches Mittel, enthaltend einen Trägerstoff und ein Azolylethylcyclopropan der Formel I

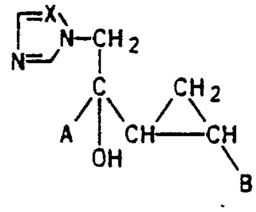

I,

in welcher
A und B gleich oder verschieden sind und jeweils $C_1$-$C_6$-Alkyl, $C_3$-$C_6$-Cycloalkyl, $C_3$-$C_6$-Cycloalkenyl, Tetrahydropyranyl, Pyridyl oder Phenyl bedeuten, wobei diese Reste einfach bis dreifach durch Halogen, Nitro, Phenoxy, Amino, Alkyl oder Halogenalkyl mit jeweils 1 bis 4 C-Atomen substituiert sein können, mit der Maßgabe, daß A und B nicht gleichzeitig Phenyl bedeuten,
X den Rest CH oder N bedeutet,
oder dessen für Pflanzen verträgliche Säureadditionssalze.

7. Verfahren zur Reduzierung des Pflanzenwachstums, dadurch gekennzeichnet, daß man eine regulatorisch wirksame Menge eines Azolylethylcyclo propans der Formel I

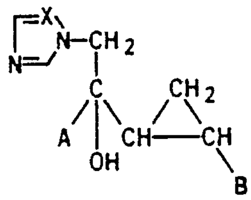

I,

in welcher
A und B gleich oder verschieden sind und jeweils $C_1$-$C_6$-Alkyl, $C_3$-$C_6$-Cycloalkyl, $C_3$-$C_6$-Cycloalkenyl, Tetrahydropyranyl, Pyridyl oder Phenyl bedeuten, wobei diese Reste einfach bis dreifach durch Halogen, Nitro, Phenoxy, Amino, Alkyl oder Halogenalkyl mit jeweils 1 bis 4 C-Atomen substituiert sein können, mit der Maßgabe, daß A und B nicht gleichzeitig Phenyl bedeuten,
X den Rest CH oder N bedeutet,
oder dessen für Pflanzen verträgliche Säureadditionssalze auf Kulturpflanzen oder deren Lebensraum einwirken läßt.

8. Verbindung der Formel I gemäß Anspruch 1, dadurch gekennzeichnet, daß A tertiär-Butyl, B 2,4-Dichlorphenyl und X N bedeutet.

9. Verbindung der Formel I gemäß Anspruch 1, dadurch gekennzeichnet, daß A tertiär-Butyl, B 4-Chlorphenyl und X N bedeutet.

10. Verbindung der Formel I gemäß Anspruch 1, dadurch gekennzeichnet, daß A Cyclopropyl, B 4-Chlorphenyl und X N bedeutet.